# EUROPEAN PATENT SPECIFICATION

(11) **EP 1 874 808 B1**
(45) Date of publication and mention of the grant of the patent: **09.02.2011**
(21) Application number: 06843685.6
(22) Date of filing: 22.12.2006
(51) Int. Cl.: C07K 14/39, C12N 15/52

(54) **CATALASE GENE AND USE THEREOF**
KATALASE-GEN UND VERWENDUNG DAVON
GÈNE DE CATALASE ET UTILISATION DE CELUI-CI

(30) Priority: 28.02.2006 JP 2006053951
(43) Date of publication of application: 09.01.2008
(60) Divisional application: 10159291.3
(73) Proprietor: Suntory Holdings Limited, Kita-ku, Osaka-shi, Osaka 530-8203 (JP)
(72) Inventor: NAKAO, Yoshihiro, Mishima-gun, Osaka 6188503 (JP); KODAMA, Yukiko, Mishima-gun, Osaka 6188503 (JP); SHIMONAGA, Tomoko, Mishima-gun, Osaka 6188503 (JP); OMURA, Fumihiko, Mishima-gun, Osaka 6188503 (JP)
(74) Representative: Vossius & Partner
(86) International application number: PCT/JP2006/326309
(87) International publication number: WO 2007/105350

(56) References cited:
- US-A1- 2004 265 862
- COHEN G ET AL: "SEQUENCE OF THE SACCHAROMYCES CEREVISIAE CTA1 GENE AND AMINO ACID SEQUENCE OF CATALASE A DERIVED FROM IT" EUROPEAN JOURNAL OF BIOCHEMISTRY, BERLIN, DE, vol. 176, no. 1, September 1988 (1988-09), pages 159-163, XP001055464 ISSN: 0014-2956
- KORCH C ET AL: "A MECHANISM FOR SULFITE PRODUCTION IN BEER AND HOW TO INCREASE SULFITE LEVELS BY RECOMBINANT GENETICS" PROCEEDINGS OF THE EUROPEAN BREWERY CONVENTION CONGRESS. LISBON, 1991, OXFORD, OUP, GB, vol. CONGRESS 23, 1991, pages 201-208, XP002066286 cited in the application

### SEQUENCE LISTING

<110> Suntory Limited
<120> Catalase gene and use thereof
<130> PCT06-0138
<150> JP2006-53951
   <151> 2006-02-28
<160> 8
<170> PatentIn version 3.3
<210> 1
   <211> 1542
   <212> DNA,
   <213> Saccharomyces sp.
<400> 1
   atgtcaggac aagaggagaa taaagtaaat tcttctgacg taagaaagga tagagttgtg      60
   acgaactcta ctggtaatcc catcaatgag ccatttgtca cccagcgtgt tggggagcac     120
   gggcctttgc ttttacaaga ttataaccta ctcgattctt tggcgcattt taacagggag     180
   aatattcctc aaagaaatcc tcacgcccac ggttctgggg ccttcggtta ttttgaagtg     240
   acagacgata ttacagatgt ttgtgggtct gccatgttta gcaagatcgg taagagaacg     300
   aagtgtctga caagattctc cactgtgggt ggtgataaag gtagtgccga tactgttcgt     360
   gacccaagag ggtttgcaac taaattctac acagaagaag gtaatttgga ttgggtctac     420
   aacaatacac ctgtattttt tatcagggat ccttcgaaat tcccccattt tatccacacg     480
   cagaagagaa acccgcaaac taatctaaga gacgctgata tgttttggga tttccttacg     540
   actccagaga atcaagtggc catccatcaa gtcatgattc tcttttcaga ccgtggtact     600
   cctgcgagct atcgtaacat gcacggatat tctggtcata cttataaatg gtcaagtaaa     660
   aacggcgatt ggcgttatgt gcaagtccat attaaaacca atcaaggggt caagaatttg     720
   actatagacg aagccactaa aatcgcaggg tccaacccag attactgcca aaaagacttg     780
   tttgaatcta tccaaagcgg taactatcca tcgtggactg tttatattca aacaatgact     840
   gaacaggagg ccaagaattt accattttcg gtctttgact tgaccaaggt atggcctcaa      900
   aagcaattcc cattacgtcg tgtaggcaaa cttgttctga atgaaaatcc actgaatttc     960
   ttcgcacaag tggaacaagc agcgtttgcc cctagtacta ctgtcccata ccaagaagcc    1020
   agtgctgatc cggtgctaca agctcgatta ttttcttatg cagatgctca cagatacaga    1080
   ctgggcccca atttccatca aatacccgtc aactgtccct atgcctccaa gttttttaac 1140
   cctgccatca gagatggccc aatgaacgta aatggaaatt ttggttcaga acctacctat 1200
   ttagccaacg acaaatcata ctcgtatatt cagcaagaaa gacctattca acaacatcaa 1260
   gaagtatgga acggacccgc tatcccttac cactgggcaa catctccagg tgatgtcgat 1320
   tatgttcaag ctaggaattt gtaccgcgtc ttagggaagc aacctggaca acaaaagaac 1380
   ctagctcaca acatcggtat ccatgtagag ggcgcctgcc ctggaatcca gcaacgggtt 1440
   tacgatatgt ttgcccgcgt agataaggga ctatctgatg cgatcaagaa agaagcagag 1500
   gcaaaacacg ctgctgaact ttcaaataac tctaagtttt ga 1542
<210> 2
   <211> 513
   <212> PRT
   <213> Saccharomyces sp.
<400> 2
   Met Ser Gly Gln Glu Glu Asn Lys Val Asn Ser Ser Asp Val Arg Lys
   1 5 10 15
   Asp Arg Val Val Thr Asn Ser Thr Gly Asn Pro Ile Asn Glu Pro Phe
   20 25 30
   Val Thr Gln Arg Val Gly Glu His Gly Pro Leu Leu Leu Gln Asp Tyr
   35 40 45
   Asn Leu Leu Asp Ser Leu Ala His Phe Asn Arg Glu Asn Ile Pro Gln
   50 55 60
   Arg Asn Pro His Ala His Gly Ser Gly Ala Phe Gly Tyr Phe Glu Val
   65 70 75 80
   Thr Asp Asp Ile Thr Asp Val Cys Gly Ser Ala Met Phe Ser Lys Ile
   85 90 95
   Gly Lys Arg Thr Lys Cys Leu Thr Arg Phe Ser Thr Val Gly Gly Asp
   100 105 110
   Lys Gly Ser Ala Asp Thr Val Arg Asp Pro Arg Gly Phe Ala Thr Lys
   115 120 125
   Phe Tyr Thr Glu Glu Gly Asn Leu Asp Trp Val Tyr Asn Asn Thr Pro
   130 135 140
   Val Phe Phe Ile Arg Asp Pro Ser Lys Phe Pro His Phe Ile His Thr
   145 150 155 160
   Gln Lys Arg Asn Pro Gln Thr Asn Leu Arg Asp Ala Asp Met Phe Trp
   165 170 175
   Asp Phe Leu Thr Thr Pro Glu Asn Gln Val Ala Ile His Gln Val Met
   180 185 190
   Ile Leu Phe Ser Asp Arg Gly Thr Pro Ala Ser Tyr Arg Asn Met His
   195 200 205
   Gly Tyr Ser Gly His Thr Tyr Lys Trp Ser Ser Lys Asn Gly Asp Trp
   210 215 220
   Arg Tyr Val Gln Val His Ile Lys Thr Asn Gln Gly Val Lys Asn Leu
   225 230 235 240
   Thr Ile Asp Glu Ala Thr Lys Ile Ala Gly Ser Asn Pro Asp Tyr Cys
   245 250 255
   Gln Lys Asp Leu Phe Glu Ser Ile Gln Ser Gly Asn Tyr Pro Ser Trp
   260 265 270
   Thr Val Tyr Ile Gln Thr Met Thr Glu Gln Glu Ala Lys Asn Leu Pro
   275 280 285
   Phe Ser Val Phe Asp Leu Thr Lys Val Trp Pro Gln Lys Gln Phe Pro
   290 295 300
   Leu Arg Arg Val Gly Lys Leu Val Leu Asn Glu Asn Pro Leu Asn Phe
   305 310 315 320
   Phe Ala Gln Val Glu Gln Ala Ala Phe Ala Pro Ser Thr Thr Val Pro
   325 330 335
   Tyr Gln Glu Ala Ser Ala Asp Pro Val Leu Gln Ala Arg Leu Phe Ser
   340 345 350
   Tyr Ala Asp Ala His Arg Tyr Arg Leu Gly Pro Asn Phe His Gln Ile
   355 360 365
   Pro Val Asn Cys Pro Tyr Ala Ser Lys Phe Phe Asn Pro Ala Ile Arg
   370 375 380
   Asp Gly Pro Met Asn Val Asn Gly Asn Phe Gly Ser Glu Pro Thr Tyr
   385 390 395 400
   Leu Ala Asn Asp Lys Ser Tyr Ser Tyr Ile Gln Gln Glu Arg Pro Ile
   405 410 415
   Gln Gln His Gln Glu Val Trp Asn Gly Pro Ala Ile Pro Tyr His Trp
   420 425 430
   Ala Thr Ser Pro Gly Asp Val Asp Tyr Val Gln Ala Arg Asn Leu Tyr
   435 440 445
   Arg Val Leu Gly Lys Gln Pro Gly Gln Gln Lys Asn Leu Ala His Asn
   450 455 460
   Ile Gly Ile His Val Glu Gly Ala Cys Pro Gly Ile Gln Gln Arg Val
   465 470 475 480
   Tyr Asp Met Phe Ala Arg Val Asp Lys Gly Leu Ser Asp Ala Ile Lys
   485 490 495
   Lys Glu Ala Glu Ala Lys His Ala Ala Glu Leu Ser Asn Asn Ser Lys
   500 505 510
   Phe
<210> 3
   <211> 1548
   <212> DNA
   <213> Saccharomyces sp.
<400> 3
   atgtcgaaat tgggacaaga aaaaaatgaa gtaaattcct ctgatgtaag agaggataga      60
   gttgtgacaa actccactgg taatccaatc aatgaaccat ttgtcaccca acgtattgga     120
   gaacatggcc ctttgctttt gcaagattat aacttaattg attctttggc tcatttcaac     180
   agggaaaata ttcctcaaag gaatccacat gctcatggtt ctggtgcctt cggctatttt     240
   gaagtaaccg atgacattac tgatatctgc gggtctgcta tgtttagtaa aattgggaaa     300
   agaacgaaat gtctaacaag attttcgact gtgggtggtg ataaaggtag tgccgacacg     360
   gttcgtgatc caaggggttt tgccaccaaa ttctacactg aagaaggtaa tttagattgg     420
   gtctacaata atacaccggt attctttatc agagaccctt ccaagttccc tcactttatc     480
   cacacacaga agagaaaccc acaaaccaac ctaagggatg ctgacatgtt ttgggatttc     540
   ctcaccactc ctgaaaatca ggtggccatt catcaagtaa tgatcctttt ttcagaccgt     600
   ggtacccctg ccaactaccg tagtatgcat ggttattctg gtcataccta taaatggtcc     660
   aataaaaacg gagattggca ttatgtgcaa gttcatatca aaaccgatca aggaataaag     720
   aatttgacca tagaagaggc taccaaaatt gcgggatcca atccagatta ctgccagcag     780
   gatttatttg aggctattca gaatggaaac tatccttcct ggacagttta tattcaaaca     840
   atgaccgaac gcgatgccaa aaaattacca ttttcagtct ttgatttgac taaagtatgg     900
   cctcaggggc aattcccttt acggcgtgtg ggtaagattg ttttgaacga gaatccactg     960
   aacttcttcg cacaggtgga acaagctgcc ttcgccccca gtaccacggt tccttaccaa    1020
   gaagcaagcg ctgatccagt attacaggcc cgtttgtttt catatgcgga tgctcataga    1080
   tacaggctag gtcctaactt ccatcaaata cccgtaaact gtccatatgc atctaaattt    1140
   ttcaatcccg ctatcagaga tggaccgatg aatgttaacg gcaacttcgg ctcagaacct    1200
   acatatttgg ccaatgataa atcgtacacg tatatccaac aggacagacc cattcaacaa    1260
   caccaagagg tatggaatgg gccagctatc ccttatcatt gggcaacatc cccaggtgat    1320
   gtagatttcg tgcaagcaag aaatctctat cgcgttttgg gtaaacaacc tggacagcaa    1380
   aagaacttgg catataacat cggcattcat gtagaaggcg cctgtcctca aatacagcag    1440
   cgcgtttatg atatgtttgc tcgtgttgat aagggactat ctgaggcaat taaaaaagta    1500
   gctgaggcaa aacatgcttc tgagctttcg agtaactcca aattttga                  1548
<210> 4
   <211> 515
   <212> PRT
   <213> Saccharomyces sp.
<400> 4
   Met Ser Lys Leu Gly Gln Glu Lys Asn Glu Val Asn Ser Ser Asp Val
   1 5 10 15
   Arg Glu Asp Arg Val Val Thr Asn Ser Thr Gly Asn Pro Ile Asn Glu
   20 25 30
   Pro Phe Val Thr Gln Arg Ile Gly Glu His Gly Pro Leu Leu Leu Gln
   35 40 45
   Asp Tyr Asn Leu Ile Asp Ser Leu Ala His Phe Asn Arg Glu Asn Ile
   50 55 60
   Pro Gln Arg Asn Pro His Ala His Gly Ser Gly Ala Phe Gly Tyr Phe
   65 70 75 80
   Glu Val Thr Asp Asp Ile Thr Asp Ile Cys Gly Ser Ala Met Phe Ser
   85 90 95
   Lys Ile Gly Lys Arg Thr Lys Cys Leu Thr Arg Phe Ser Thr Val Gly
   100 105 110
   Gly Asp Lys Gly Ser Ala Asp Thr Val Arg Asp Pro Arg Gly Phe Ala
   115 120 125
   Thr Lys Phe Tyr Thr Glu Glu Gly Asn Leu Asp Trp Val Tyr Asn Asn
   130 135 140
   Thr Pro Val Phe Phe Ile Arg Asp Pro Ser Lys Phe Pro His Phe Ile
   145 150 155 160
   His Thr Gln Lys Arg Asn Pro Gln Thr Asn Leu Arg Asp Ala Asp Met
   165 170 175
   Phe Trp Asp Phe Leu Thr Thr Pro Glu Asn Gln Val Ala Ile His Gln
   180 185 190
   Val Met Ile Leu Phe Ser Asp Arg Gly Thr Pro Ala Asn Tyr Arg Ser
   195 200 205
   Met His Gly Tyr Ser Gly His Thr Tyr Lys Trp Ser Asn Lys Asn Gly
   210 215 220
   Asp Trp His Tyr Val Gln Val His Ile Lys Thr Asp Gln Gly Ile Lys
   225 230 235 240
   Asn Leu Thr Ile Glu Glu Ala Thr Lys Ile Ala Gly Ser Asn Pro Asp
   245 250 255
   Tyr Cys Gln Gln Asp Leu Phe Glu Ala Ile Gln Asn Gly Asn Tyr Pro
   260 265 270
   Ser Trp Thr Val Tyr Ile Gln Thr Met Thr Glu Arg Asp Ala Lys Lys
   275 280 285
   Leu Pro Phe Ser Val Phe Asp Leu Thr Lys Val Trp Pro Gln Gly Gln
   290 295 300
   Phe Pro Leu Arg Arg Val Gly Lys Ile Val Leu Asn Glu Asn Pro Leu
   305 310 315 320
   Asn Phe Phe Ala Gln Val Glu Gln Ala Ala Phe Ala Pro Ser Thr Thr
   325 330 335
   Val Pro Tyr Gln Glu Ala Ser Ala Asp Pro Val Leu Gln Ala Arg Leu
   340 345 350
   Phe Ser Tyr Ala Asp Ala His Arg Tyr Arg Leu Gly Pro Asn Phe His
   355 360 365
   Gln Ile Pro Val Asn Cys Pro Tyr Ala Ser Lys Phe Phe Asn Pro Ala
   370 375 380
   Ile Arg Asp Gly Pro Met Asn Val Asn Gly Asn Phe Gly Ser Glu Pro
   385 390 395 400
   Thr Tyr Leu Ala Asn Asp Lys Ser Tyr Thr Tyr Ile Gln Gln Asp Arg
   405 410 415
   Pro Ile Gln Gln His Gln Glu Val Trp Asn Gly Pro Ala Ile Pro Tyr
   420 425 430
   His Trp Ala Thr Ser Pro Gly Asp Val Asp Phe Val Gln Ala Arg Asn
   435 440 445
   Leu Tyr Arg Val Leu Gly Lys Gln Pro Gly Gln Gln Lys Asn Leu Ala
   450 455 460
   Tyr Asn Ile Gly Ile His Val Glu Gly Ala Cys Pro Gln Ile Gln Gln
   465 470 475 480
   Arg Val Tyr Asp Met Phe Ala Arg Val Asp Lys Gly Leu Ser Glu Ala
   485 490 495
   Ile Lys Lys Val Ala Glu Ala Lys His Ala Ser Glu Leu Ser Ser Asn
   500 505 510
   Ser Lys Phe
   515
<210> 5
   <211> 21
   <212> DNA
   <213> Artificial Sequence
<220>
   <223> Primer
<400> 5
   gagctcatgt caggacaaga g 21
<210> 6
   <211> 23
   <212> DNA
   <213> Artificial Sequence
<220>
   <223> Primer
<400> 6
   gcggccgctg acttctttac ttc 23
<210> 7
   <211> 36
   <212> DNA
   <213> Artificial Sequence
<220>
   <223> Primer
<400> 7
   gagctcatgt cgaaattggg ocaagaaaaa aatgaa 36
<210> 8
   <211> 38
   <212> DNA
   <213> Artificial Sequence
<220>
   <223> Primer
<400> 8
   gcggccgctc aaaatttgga gttactcgaa agctcaga 38

## Description

### TECHNICAL FIELD

The present invention relates to a gene encoding catalase and use thereof in particular; a brewery yeast for producing alcoholic beverages with superior flavor, alcoholic beverages produced with said yeast, and a method for producing said beverages. More particularly, the present invention relates to a yeast, whose capability of producing sulfite that contribute to stability of flavor in products, is enhanced by amplifying expression level of CTAl gene encoding Ctalp that is a catalase in a brewery yeast, especially non-ScCTAl gene or ScCTAl gene specific to a lager brewing yeast, and to a method for producing alcoholic beverages with said yeast.

### BACKGROUND ART

Sulfite has been known as a compound having high anti-oxidative activity, and thus has been widely used in the fields of food, beverages, pharmaceutical products or the like (for example, Japanese Patent Application Laid-Open Nos. H06-040907 and 2000-093096). In alcoholic beverages, sulfite has been used as an anti-oxidant. For Example, because sulfite plays an important role in quality maintenance of wine that needs long-term maturation, addition of up to 350 ppm (parts per million) of residual concentration is permitted by the Ministry of Health, Welfare and Labor in Japan. Further, it is also known that shelf life (quality maintained period) varies depending upon sulfite concentration in a product in beer brewing. Thus, it is quite important to increase the content of this compound from the viewpoint of flavor stability or the like.

The easiest way to increase the sulfite content in a product is addition of sulfite. However, sulfite is treated as a food additive, resulting in some problems such as constraint of product development and negative images of food additives of consumers.

Methods of increasing sulfite content in a fermentation liquor during brewing process include (1) a method based on process control, and (2) a method based on breeding of yeast. In the method based on a process control, since the amount of sulfite produced is in inverse proportion to the amount of initial oxygen supply, supplied amount of oxygen is reduced to increase amount of sulfite produced and to prevent oxidation.

On the other hand, gene manipulation techniques are used in the method based on breeding of yeast. Yeast biosynthesizes sulfur-containing compounds which are required for its biological activity. Sulfite is produced as an intermediate in the biosynthesis of sulfur-containing compounds. Thus, amount of sulfite in products can be increased by utilizing the ability of yeast without adding sulfite from outside.

The MET3 and MET14 are genes encoding reductases participating in steps which are involved in biosynthesis of sulfite from sulfate ion taken from culture medium. Korch et al. attempted to increase a sulfite-producing capability of yeasts by increasing expression level of the two genes, and found that MET14 is more effective (C. Korch et al., Proc. Eur. Brew. Conv. Conger., Lisbon, 201-208, 1991). Also, Hansen et al. attempted to increase production amount of sulfite by disrupting MET 10 gene encoding a sulfite ion reductase to prevent reduction of sulfite produced (J. Hansen et al., Nature Biotech., 1587-1591, 1996). On the other hand, however, delay in fermentation or increase in acetaldehyde and 1-propanol, which are undesirable flavor ingredients, are also observed.

Further, Fujimura et al. attempted to increase sulfite content in beer by increasing expression level of a non-ScSSU1 gene unique to a lager brewing yeast among SSU1 genes encoding sulfite ion efflux pump of yeast to promote excretion of sulfite to outside the yeast cells (Fujimura et al., Abstract of 2003 Annual Conference of the Japan Society for Bioscience, Biotechnology and Agrochem., 159, 2003).

### DISCLOSURE OF INVENTION

As mentioned above, the easiest way to increase sulfite content in a product is addition of sulfite. However, it is desirable to minimize use of food additives in view of recent consumers' preference, i.e., avoidance of food additives and preference of natural materials. Thus, it is desirable to achieve sulfite content which is effective level for flavor stability by use of biological activity of yeast itself without adding sulfite from outside. However, the method based on a process control as described above may not be practical since shortage of oxygen may cause decrease in growth rate of yeasts, resulting in delay in fermentation and quality loss.

Further, in breeding of yeast using gene manipulation techniques, there is a report stating that ten times or more sulfite content than parent strain was achieved (J. Hansen et al., Nature Biotech., 1587-1591, 1996). However, there are problems such as delay in fermentation and increase of undesirable flavor ingredients such as acetaldehyde and 1-propanol. Thus, there are problems with the yeast for practical use. Thus, there has been a need for a method for breeding yeast capable of producing sufficient amount of sulfite without impairing the fermentation rates and quality of the products.

To solve the problems described above, the present inventors made extensive studies, and as a result succeeded in identifying and isolating a gene encoding a catalase from a lager brewing yeast. Moreover, a yeast in which the obtained gene was transformed and expressed was produced to confirm increase in production of sulfite, thereby completing the present invention.

Thus, the present invention relates to a catalase gene existing in a lager brewing yeast, to a protein encoded by said gene, to a transformed yeast in which the expression of said gene is controlled, to a method for controlling the amount of sulfite produced in a product by using a yeast in which the expression of said gene is controlled. More specifically, the present invention provides the following polynucleotides, a vector comprising said polynucleotide, a transformed yeast introduced with said vector, a method for producing alcoholic beverages by using said transformed yeast, and the like.
(1) A polynucleotide selected from the group consisting of:
   (a) a polynucleotide comprising a polynucleotide consisting of the nucleotide sequence of SEQ ID NO:1;
   (b) a polynucleotide comprising a polynucleotide encoding a protein consisting of the amino acid sequence of SEQ ID NO:2;
   (c) a polynucleotide comprising a polynucleotide encoding a protein consisting of the amino acid sequence of SEQ ID NO:2 with one to 5 amino acids thereof being deleted, substituted, inserted and/or added, and having a catalase activity;
   (d) a polynucleotide comprising a polynucleotide encoding a protein having an amino acid sequence having 99 % or higher identity with the amino acid sequence of SEQ ID NO: 2, and having a catalase activity.
(2) The polynucleotide of (1) above comprising a polynucleotide consisting of SEQ ID NO: 1.
(3) The polynucleotide of (1) above comprising a polynucleotide encoding a protein consisting of SEQ ID NO: 2.
(4) The polynucleotide of any one of (1) to (3) above, wherein the polynucleotide is DNA.
(5) A protein encoded by the polynucleotide of any one of (1) to (4) above.
(6) A vector comprising the polynucleotide of any one of (1) to (4) above.

The present invention also discloses a vector as defined in (6) above which comprises the expression cassette comprising the following components:
(x) a promoter that can be transcribed in a yeast cell;
(y) any of the polynucleotides described in (1) to (4) above linked to the promoter in a sense or antisense direction; and
(z) a signal that can function in a yeast with respect to transcription termination and polyadenylation of a RNA molecule.

(7) A yeast, wherein the vector of (7) above is introduced.
(8) The yeast of (7) above, wherein a sulfite-producing capability is enhanced.
(9) The yeast of (8) above, wherein a sulfite-producing capability is enhanced by increasing an expression level of the protein of (5) above.
(10) A method for producing an alcoholic beverage comprising culturing the yeast of any one of (7) to (9) above.
(11) The method for producing an alcoholic beverage of (10) above, wherein the brewed alcoholic beverage is a malt beverages.
(12) The method for producing an alcoholic beverage of (10) above, wherein the brewed alcoholic beverage is wine.
(13) A method for assessing a test yeast for its sulfite-producing capability, comprising using a primer or a probe designed based on a nucleotide sequence of a gene having the nucleotide sequence of SEQ ID NO: 1.

Also disclosed is a method for selecting a yeast having an enhanced sulfite-producing capability by using the method described in (13) above as well as a method for producing an alcoholic beverage (for example, beer) by using the yeast selected with the aforementioned method.
(14) A method for assessing a test yeast for its sulfite-producing capability, comprising: culturing a test yeast; and measuring an expression level of a gene having the nucleotide sequence of SEQ ID NO: 1.
   Also disclosed is a method for selecting a yeast having a high sulfite-producing capability, which comprises assessing a test yeast by the method described in (14) above and selecting a yeast having a high expression level of a gene encoding a protein having a catalase activity as well as a method for producing an alcoholic beverage (for example, beer) by using the yeast selected with the aforementioned method.
   Also disclosed is a method for selecting a yeast, comprising: culturing test yeasts; quantifying the protein according to (5) or measuring an expression level of a gene having the nucleotide sequence of SEQ ID NO: 1, - and selecting a test yeast having said protein amount or said gene expression level according to a target sulfite-producing capability.
(15) The present invention in particular provides a method for selecting a yeast comprising: culturing a reference yeast and test yeasts; measuring an expression level of a gene having the nucleotide
   sequence of SEQ ID NO: 1 in each yeast; and selecting a test yeast having the gene expressed higher than that in the reference yeast.
(16) The present invention also provides a method for selecting a yeast comprising: culturing a reference yeast and test yeasts; quantifying the protein according to (5) above in each yeast; and selecting a test yeast having said protein in a larger amount than that in the reference yeast.
(17) A method for producing an alcoholic beverage comprising: conducting fermentation for producing an alcoholic beverage using the yeast according to any one of (7) to (9) above; and adjusting sulfite concentration.

According to the method for producing alcoholic beverages of the present invention, the content of sulfite which has an anti-oxidative activity in products can be increased so that alcoholic beverages which have superior stability of flavor and longer shelf life can be produced.

### BRIEF DESCRIPTION OF DRAWINGS

Figure 1 shows the cell growth with time upon beer fermentation test. The horizontal axis represents fermentation time while the vertical axis represents optical density at 660 nm (OD660).
Figure 2 shows the extract consumption with time upon beer fermentation test. The horizontal axis represents fermentation time while the vertical axis represents apparent extract concentration (w/w%).
Figure 3 shows the expression behavior of non-ScCTAl gene in yeasts upon beer fermentation test. The horizontal axis represents fermentation time while the vertical axis represents the brightness of detected signal.
Figure 4 shows the cell growth with time upon beer fermentation test using the non-SCCTAl -highly expressed strain. The horizontal axis represents fermentation time while the vertical axis represents optical density at 660 nm (OD660).
Figure 5 shows the extract consumption with time upon beer fermentation test using the non-ScCTAl-highly expressed strain. The horizontal axis represents fermentation time while the vertical axis represents apparent extract concentration (w/w%).
Figure 6 shows sulfite concentration in the fermentation broth (at the completion of fermentation) during beer fermentation test using the non-ScCTAl-highly expressed strain.
Figure 7 shows the cell growth with time upon beer fermentation test. The horizontal axis represents fermentation time while the vertical axis represents optical density at 660 nm (OD660).
Figure 8 shows the extract consumption with time upon beer fermentation test. The horizontal axis represents fermentation time while the vertical axis represents apparent extract concentration (w/w%).
Figure 9 shows the expression behavior of ScCTAl gene in yeasts upon beer fermentation test. The horizontal axis represents fermentation time while the vertical axis represents the brightness of detected signal.
Figure 10 shows the cell growth with time upon beer fermentation test using the ScCTAl-highly expressed strain. The horizontal axis represents fermentation time while the vertical axis represents optical density at 660 nm (OD660).
Figure 11 shows the extract consumption with time upon beer fermentation test using the ScCTAl-highly expressed strain. The horizontal axis represents fermentation time while the vertical axis represents apparent extract concentration (w/w%).
Figure 12 shows sulfate concentration in the fermentation broth (at the completion of fermentation) during beer fermentation test using the ScCTAl -highly expressed strain.

### BEST MODES FOR CARRYING OUT THE INVENTION

The present inventors isolated and identified non-ScCTAl gene encoding a protein having a catalase activity unique to lager brewing yeast based on the lager brewing yeast genome information mapped according to the method disclosed in Japanese Patent Application Laid-Open No. 2004-283169. The nucleotide sequence of the gene is represented by SEQ ID NO: 1. Further, an amino acid sequence of a protein encoded by the gene is represented by SEQ ID NO: 2. Furthermore, the present inventors isolated and identified ScCTAl gene encoding a protein having a catalase activity unique to lager brewing yeast. The nucleotide sequence of the gene is represented by SEQ ID NO: 3. Further, an amino acid sequence of a protein encoded by the gene is represented by SEQ ID NO: 4.

### 1. Polynucleotide of the invention

First of all, the present invention provides (a) a polynucleotide comprising a polynucleotide of the nucleotide -sequence of SEQ ID NO: 1 and (b) a polynucleotide comprising a polynucleotide encoding a protein of the amino acid sequence of SEQ ID NO:2. The polynucleotide can be DNA or RNA.

The target polynucleotide of the present invention is not limited to the polynucleotide encoding a protein having a catalase activity derived from lager brewing yeast and may include other polynucleotides encoding proteins having equivalent functions to said protein. Proteins with equivalent functions include, for example, (c) a protein of an amino acid sequence of SEQ ID NO: 2 with one to 5 amino acids thereof being deleted, substituted, inserted and/or added and having a catalase activity.

Such proteins include a protein consisting of an amino acid sequence of SEQ ID NO: 2 with, for example, 1 to 5, 1 to 4, 1 to 3, 1 to 2, or 1 amino acid residues thereof being deleted, substituted, inserted and/or added and having a catalase activity. In general, the number of deletions, substitutions, insertions, and/or additions is preferably smaller. In addition, such proteins include (d) a protein having an amino acid sequence with 99% or higher, 99. 1 % or higher, 99.2% or higher, 99.3% or higher, 99.4% or higher, 99.5% or higher, 99.6% or higher, 99.7% or higher, 99.8% or higher, or 99.9% or higher identity with the amino acid sequence of SEQ ID NO: 2 and having a catalase activity. In general, the percentage identity is preferably higher.

The catalase activity can be assessed, for example by, a method of Osorio et al., Archives of Microbiology, 181 (3), 231-236 (2004).

Furthermore, the present invention also discloses (e) a polynucleotide comprising a polynucleotide which hybridizes to a polynucleotide consisting of a nucleotide sequence complementary to the nucleotide sequence of SEQ ID NO: 1 under stringent conditions and which encodes a protein having a catalase activity, and (f) a polynucleotide comprising a polynucleotide which hybridizes to a polynucleotide complementary to a nucleotide sequence of encoding a protein of SEQ ID NO: 2 under stringent conditions, and which encodes a protein having a catalase activity.

Herein, "a polynucleotide that hybridizes under stringent conditions" refers to nucleotide sequence, such as a DNA, obtained by a colony hybridization technique, a plaque hybridization technique, a southern hybridization technique or the like using all or part of polynucleotide of a nucleotide sequence complementary to the nucleotide sequence of SEQ ID NO: 1 or polynucleotide encoding the amino acid sequence of SEQ ID NO: 2 as a probe. The hybridization method may be a method described, for example, in Molecular Cloning 3rd Ed., Current Protocols in Molecular Biology, John Wiley & Sons 1987-1997.

The term "stringent conditions" as used herein may be any of low stringency conditions, moderate stringency conditions or high stringency conditions. "Low stringency conditions" are, for example, 5 x SSC, 5 x Denhardt's solution, 0.5% SDS, 50% formamide at 32°C. "Moderate stringency conditions" are, for example, 5 x SSC, 5 x Denhardt's solution, 0.5% SDS, 50% formamide at 42°C. "High stringency conditions" are, for example, 5 x SSC, 5 x Denhardt's solution, 0.5% SDS, 50% formamide at 50°C. Under these conditions, a polynucleotide, such as a DNA, with higher homology is expected to be obtained efficiently at higher temperature, although multiple factors are involved in hybridization stringency including temperature, probe concentration, probe length, ionic strength, time, salt concentration and others, and one skilled in the art may appropriately select these factors to realize similar stringency.

When a commercially available kit is used for hybridization, for example, Alkphos Direct Labeling Reagents (Amersham Pharmacia) may be used. In this case, according to the attached protocol, after incubation with a labeled probe overnight, the membrane is washed with a primary wash buffer containing 0.1% (w/v) SDS at 55°C, thereby detecting hybridized polynucleotide, such as DNA.

Polynucleotides that can be hybridized include polynucleotides having 99% or higher, 99.1% or higher, 99.2% or higher, 99.3% or higher, 99.4% or higher, 99.5% or higher, 99.6% or higher, 99.7% or higher, 99.8% or higher or 99.9% or higher identity to polynucleotide encoding the amino acid sequence of SEQ ID NO: 2 as calculated by homology search software, such as FASTA and BLAST using default parameters.

Identity between amino acid sequences or nucleotide sequences may be determined using algorithm BLAST by Karlin and Altschul (Proc. Natl. Acad. Sci. USA, 87: 2264-2268, 1990; Proc. Natl. Acad. Sci. USA, 90: 5873, 1993). Programs called BLASTN and BLASTX based on BLAST algorithm have been developed (Altschul SF et al., J. Mol. Biol. 215: 403; 1990). When a nucleotide sequence is sequenced using BLASTN, the parameters are, for example, score = 100 and word length = 12. When an amino acid sequence is sequenced using BLASTX, the parameters are, for example, score = 50 and word length = 3. When BLAST and Gapped BLAST programs are used, default parameters for each of the programs are employed.

### 2. Protein of the present invention

The present invention also provides proteins encoded by any of the polynucleotides (a) to (d) above. A preferred protein of the present invention comprises an amino acid sequence of SEQ ID NO:2 with one to 5 amino acids thereof being deleted, substituted, inserted and/or added, and has a catalase activity.

Such protein includes those having an amino acid sequence of SEQ ID NO: 2 with amino acid residues thereof of the number mentioned above being deleted, substituted, inserted and/or added and having a catalase activity. In addition, such protein includes those having homology as described above with the amino acid sequence of SEQ ID NO: 2 and having a catalase activity.

Such proteins may be obtained by employing site-directed mutation described, for example, in Molecular Cloning 3rd Ed., Current Protocols in Molecular Biology, Nuc. Acids. Res., 10: 6487 (1982), Proc. Natl. Acad. Sci. USA 79: 6409 (1982), Gene 34: 315 (1985), Nuc. Acids. Res., 13: 4431 (1985), Proc. Natl. Acad. Sci. USA 82: 488 (1985).

Deletion, substitution, insertion and/or addition of one to 5 amino acid residues in an amino acid sequence of the protein of the invention means that one to 5 amino acid residues are deleted, substituted, inserted and/or added at any one or more positions in the same amino acid sequence. Two or more types of deletion, substitution, insertion and/or addition may occur concurrently.

Hereinafter, examples of mutually substitutable amino acid residues are enumerated. Amino acid residues in the same group are mutually substitutable. The groups are provided below.

Group A: leucine, isoleucine, norleucine, valine, norvaline, alanine, 2-aminobutanoic acid, methionine, o-methylserine, t-butylglycine, t-butylalanine, cyclohexylalanine; Group B: asparatic acid, glutamic acid, isoasparatic acid, isoglutamic acid, 2-aminoadipic acid, 2-aminosuberic acid; Group C: asparagine, glutamine; Group D: lysine, arginine, ornithine, 2,4-diaminobutanoic acid, 2,3-diaminopropionic acid; Group E: proline, 3-hydroxyproline, 4-hydroxyproline; Group F: serine, threonine, homoserine; and Group G: phenylalanine, tyrosine.

The protein of the present invention may also be produced by chemical synthesis methods such as Fmoc method (fluorenylmethyloxycarbonyl method) and tBoc method (t-butyloxycarbonyl method). In addition, peptide synthesizers available from, for example, Advanced ChemTech, PerkinElmer, Pharmacia, Protein Technology Instrument, Synthecell-Vega, PerSeptive, Shimazu Corp. can also be used for chemical synthesis.

### 3. Vector of the invention and yeast transformed with the vector

The present invention then provides a vector comprising the polynucleotide described above. The vector of the present invention is directed to a vector including any of the polynucleotides (DNA) described in (a) to (d) above. Generally, the vector of the present invention comprises an expression cassette including as components (x) a promoter that can transcribe in a yeast cell; (y) a polynucleotide (DNA) described in any of (a) to (d) above that is linked to the promoter in sense or antisense direction; and (z) a signal that functions in the yeast with respect to transcription termination and polyadenylation of RNA molecule.

A vector introduced in the yeast may be any of a multicopy type (YEp type), a single copy type (YCp type), or a chromosome integration type (YIp type). For example, YEp24 (J. R. Broach et al., Experimental Manipulation of Gene Expression, Academic Press, New York, 83, 1983) is known as a YEp type vector, YCp50 (M. D. Rose et al., Gene 60: 237, 1987) is known as a YCp type vector, and YIp5 (K. Struhl et al., Proc. Natl. Acad. Sci. USA, 76: 1035, 1979) is known as a YIp type vector, all of which are readily available.

Promoters/terminators for adjusting gene expression in yeast may be in any combination as long as they function in the brewery yeast and they have no influence on the concentration of constituents in fermentation broth For example, a promoter of glyceraldehydes 3-phosphate dehydrogenase gene (TDH3), or a promoter of 3-phosphoglycerate kinase gene (PGKl) may be used. These genes have previously been cloned, described in detail, for example, in M. F. Tuite et al., EMBO J., 1, 603 (1982), and are readily available by known methods.

Since an auxotrophy marker cannot be used as a selective marker upon transformation for a brewery yeast, for example, a geneticin-resistant gene (G418r), a copper-resistant gene (CUPl) (Marin et al., Proc. Natl. Acad. Sci. USA, 81, 337 1984) or a cerulenin-resistant gene (fas2m, PDR4) (Junji Inokoshi et al., Biochemistry, 64, 660, 1992; and Hussain et al., Gene, 101: 149, 1991, respectively) may be used.

A vector constructed as described above is introduced into a host yeast. Examples of the host yeast include any yeast that can be used for brewing, for example, brewery yeasts for beer, wine and sake. Specifically, yeasts such as genus *Saccharomyces* may be used. According to the present invention, a lager brewing yeast, for example, *Saccharomyces pastorianus* W34/70, *Saccharomyces carlsbergensis* NCYC453 or NCYC456, or *Saccharomyces cerevisiae* NBRC1951, NBRC1952, NBRC1953 or NBRC1954 may be used. In addition, wine yeasts such as wine yeasts #1, 3 and 4 from the Brewing Society of Japan, and sake yeasts such as sake yeast #7 and 9 from the Brewing Society of Japan may also be used but not limited thereto. In the present invention, lager brewing yeasts such as *Saccharomyces pastorianus* may be used preferably.

A yeast transformation method may be a generally used known method. For example, methods that can be used include but not limited to an electroporation method (Meth. Enzym., 194: 182 (1990)), a spheroplast method (Proc. Natl. Acad. Sci. USA, 75: 1929(1978)), a lithium acetate method (J. Bacteriology, 153: 163 (1983)), and methods described in Proc. Natl. Acad. Sci. USA, 75: 1929 (1978), Methods in Yeast Genetics, 2000 Edition: A Cold Spring Harbor Laboratory Course Manual.

More specifically, a host yeast is cultured in a standard yeast nutrition medium (e.g., YEPD medium (Genetic Engineering. Vol. 1, Plenum Press, New York, 117(1979)), etc.) such that OD600 nm will be 1 to 6. This culture yeast is collected by centrifugation, washed and pre-treated with alkali ion metal ion, preferably lithium ion at a concentration of about 1 to 2 M. After the cell is left to stand at about 30°C for about 60 minutes, it is left to stand with DNA to be introduced (about 1 to 20 µg) at about 30°C for about another 60 minutes. Polyethyleneglycol, preferably about 4,000 Dalton of polyethyleneglycol, is added to a final concentration of about 20% to 50%. After leaving at about 30°C for about 30 minutes, the cell is heated at about 42°C for about 5 minutes. Preferably, this cell suspension is washed with a standard yeast nutrition medium, added to a predetermined amount of fresh standard yeast nutrition medium and left to stand at about 30°C for about 60 minutes. Thereafter, it is seeded to a standard agar medium containing an antibiotic or the like as a selective marker to obtain a transformant.

Other general cloning techniques may be found, for example, in Molecular Cloning 3rd Ed., and Methods in Yeast Genetics, A Laboratory Manual (Cold Spring Harbor Laboratory Press, Cold Spring Harbor, NY).

### 4. Method of producing alcoholic beverages according to the present invention and alcoholic beverages produced by the method

The vector of the present invention described above is introduced into a yeast suitable for brewing a target alcoholic product. This yeast can be used to increase content of sulfite of desired alcoholic beverages with superior stability of flavor. In addition, yeasts to be selected by the yeast assessment method of the present invention described below can also be used. The target alcoholic beverages include, for example, but not limited to beer, sparkling liquor (*happoushu*) such as a beer-taste beverage, wine, sake and the like.

In order to produce these alcoholic beverages, a known technique can be used except that a brewery yeast obtained according to the present invention is used in the place of a parent strain. Since materials, manufacturing equipment, manufacturing control and the like may be exactly the same as the conventional ones, there is no need of increasing the cost for producing alcoholic beverages with increased content of sulfite. Thus, according to the present invention, alcoholic beverages with superior stability of flavor can be produced using the existing facility without increasing the cost.

### 5. Yeast assessment method of the invention

The present invention relates to a method for assessing a test yeast for its capability of producing sulfite by using a primer or a probe designed based on a nucleotide sequence of a gene having the nucleotide sequence of SEQ ID NO:1 and encoding a protein having a catalase activity. General techniques for such assessment method are known and are described in, for example, WO01/04514, Japanese Laid-Open Patent Application No. 8-205900 or the like. This assessment method is described in below.

First, genome of a test yeast is prepared. For this preparation, any known method such as Hereford method or potassium acetate method may be used (e.g., Methods in Yeast Genetics, Cold Spring Harbor Laboratory Press, 130(1990)). Using a primer or a probe designed based on a nucleotide sequence (preferably, ORF sequence) of the gene encoding a protein having a catalase activity, the existence of the gene or a sequence specific to the gene is determined in the test yeast genome obtained. The primer or the probe may be designed according to a known technique.

Detection of the gene or the specific sequence may be carried out by employing a known technique. For example, a polynucleotide including part or all of the specific sequence or a polynucleotide including a nucleotide sequence complementary to said nucleotide sequence is used as one primer, while a polynucleotide including part or all of the sequence upstream or downstream from this sequence or a polynucleotide including a nucleotide sequence complementary to said nucleotide sequence, is used as another primer to amplify a nucleic acid of the yeast by a PCR method, thereby determining the existence of amplified products and molecular weight of the amplified products. The number of bases of polynucleotide used for a primer is generally 10 base pairs (bp) or more, and preferably 15 to 25 bp. In general, the number of bases between the primers is suitably 300 to 2000 bp.

The reaction conditions for PCR are not particularly limited but may be, for example, a denaturation temperature of 90 to 95°C, an annealing temperature of 40 to 60°C, an elongation temperature of 60 to 75°C, and the number of cycle of 10 or more. The resulting reaction product may be separated, for example, by electrophoresis using agarose gel to determine the molecular weight of the amplified product. This method allows prediction and assessment of the capability of producing sulfite of the yeast as determined by whether the molecular weight of the amplified product is a size that contains the DNA molecule of the specific part. In addition, by analyzing the nucleotide sequence of the amplified product, the capability may be predicted and/or assessed more precisely.

Moreover, in the present invention, a test yeast is cultured to measure an expression level of the gene having the nucleotide sequence of SEQ ID NO: 1 and encoding a protein having a catalase activity to assess the test yeast for its capability of producing sulfite. In measuring an expression level of the gene encoding a protein having a catalase activity, the test yeast is cultured, and then mRNA or a protein resulting from the gene encoding a protein having a catalase activity, is quantified. The quantification of mRNA or protein may be carried out by employing a known technique. For example, mRNA may be quantified, by Northern hybridization or quantitative RT-PCR, while protein may be quantified, for example, by Western blotting (Current Protocols in Molecular Biology, John Wiley & Sons 1994-2003). Further, expression level of the above-identified gene of the test yeast may be projected by measuring sulfite concentration of fermentation broth obtained after fermentation of the test yeast.

Furthermore, test yeasts are cultured and expression levels of the gene having the nucleotide sequence of SEQ ID NO: 1 and encoding a protein having a catalyse activity are measured to select a test yeast with the gene expression level according to the target catalase activity, thereby selecting a yeast favorable for brewing desired alcoholic beverages. In addition, a reference yeast and a test yeast may be cultured so as to measure and compare the expression level of the gene in each of the yeasts, thereby selecting a favorable test yeast. More specifically, for example, a reference yeast and one or more test yeasts are cultured and an expression level of the gene having the nucleotide sequence of SEQ ID NO: 1 and encoding a protein having a catalase activity, is measured in each yeast. By selecting a test yeast with the gene expressed higher than that in the reference yeast, a yeast suitable for brewing alcoholic beverages can be selected.

Alternatively, test yeasts are cultured and a yeast with a higher catalase activity is selected, thereby selecting a yeast suitable for brewing desired alcoholic beverages.

In these cases, the test yeasts or the reference yeast may be, for example, a yeast introduced with the vector of the invention, an artificially mutated yeast or a naturally mutated yeast. The catalase activity can be assessed, for example by, a method of Osorio et al., Archives of Microbiology, 181(3), 231-236 (2004). The mutation treatment may employ any methods including, for example, physical methods such as ultraviolet irradiation and radiation irradiation, and chemical methods associated with treatments with drugs such as EMUS (ethylmethane sulphonate) and N-methyt-N-nitrosoguanidine (see, e.g., Yasuji Oshima Ed., Biochemistry Experiments vol. 39, Yeast Molecular Genetic Experiments, pp. 67-75, JSSP).

In addition, examples of yeasts used as the reference yeast or the test yeasts include any yeasts that can be used for brewing, for example, brewery yeasts for beer, wine, sake and the like. More specifically, yeasts such as genus *Saccharomyces* may be used (e.g., *S. pastiorianus*, *S. cerevisiae,* and *S*. *carlsbergensis*). According to the present invention, a lager brewing yeast, for example, *Saccharomyces pastorianus* W34/70; *Saccharomyces carlsbergensis* NCYC453 or NCYC456; or *Saccharomyces cerevisiae* NBRC1951, NBRC1952, NBRC1953 or NBRC1954 may be used. Further, whisky yeasts such as *Saccharomyces cerevisiae* NCYC90; wine yeasts such as wine yeasts #1, 3 and 4 from the Brewing Society of Japan; and sake yeasts such as sake yeast #7 and 9 from the Brewing Society of Japan may also be used but not limited thereto. In the present invention, lager brewing yeasts such as *Saccharomyces pastorianus* may preferably be used. The reference yeast and the test yeasts may be selected from the above yeasts in any combination.

### EXAMPLES

Hereinafter, the present invention will be described in more detail with reference to working examples. The present invention, however, is not limited to the examples described below.

### Example 1: Cloning of Gene Encoding Catalase of Laser Brewing Yeast (non-ScCTA1)

A gene encoding a catalase (non-ScCTA1 gene; SEQ ID NO: 1) specific to a lager brewing yeast was found, as a result of a search utilizing the comparison database described in Japanese Patent Application Laid-Open No. 2004-283169. Based on the acquired nucleotide sequence information, primers non-ScCTA1_for (SEQ ID NO: 5) and non-ScCTA1_rv (SEQ ID NO: 6) were designed to amplify the full-length genes, respectively. PCR was carried out using chromosomal DNA of a genome sequencing strain, *Saccharomyces pastorianus* Weihenstephan 34/70 strain (also sometimes referred to as "W34/70 strain"), as a template to obtain DNA fragments including the full-length gene of non-ScCTA1.

The thus-obtained non-ScCTA1 gene fragment was inserted into pCR2.1-TOPO vector (manufactured by Invitrogen Corporation) by TA cloning. The nucleotide sequences of non-ScCTA1 gene were analyzed according to Sanger's method (F. Sanger, Science, 214: 1215, 1981) to confirm the nucleotide sequence.

### Example 2: Analysis of Expression of non-ScCTA1 Gene during Beer Fermentation

A beer fermentation test was conducted using a lager brewing yeast, *Saccharomyces pastorianus* 34/70 strain and then mRNA extracted from yeast cells during fermentation was analyzed by a yeast DNA microarray.

| | |
|---|---|
| Wort extract concentration | 12.69% |
| Wort content | 70 L |
| Wort dissolved oxygen concentration | 8.6 ppm |
| Fermentation temperature | 15°C |
| Yeast pitching rate | 12.8×10⁶ cells/mL |

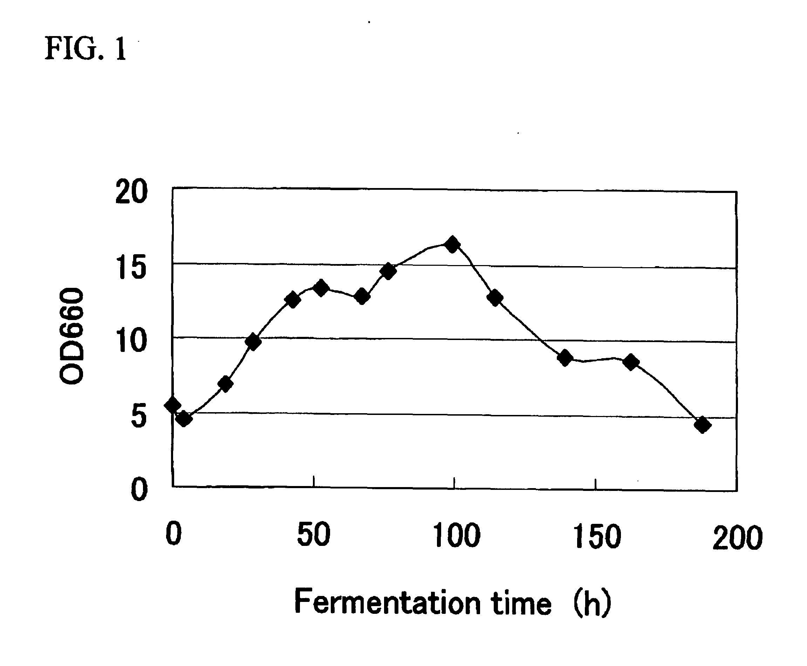

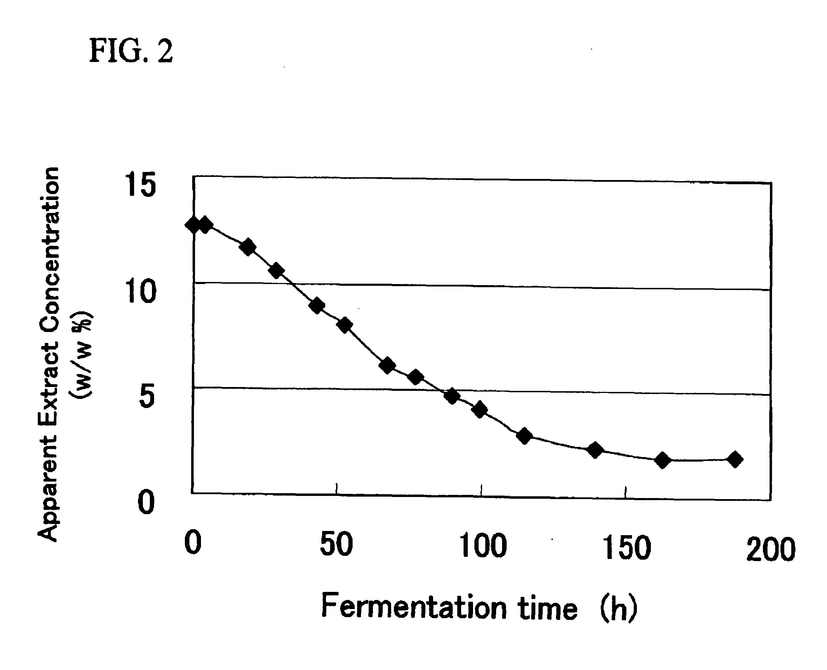

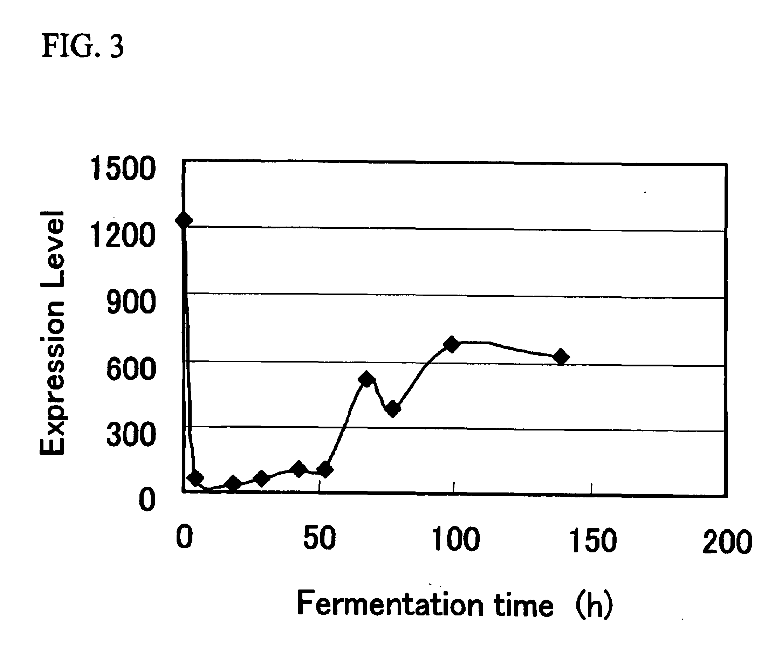

Sampling of fermentation liquid was performed with time, and variation with time of yeast growth amount (Fig. 1) and apparent extract concentration (Fig. 2) was observed. Simultaneously, yeast cells were sampled to prepare mRNA, and the prepared mRNA was labeled with biotin and was hybridized to a beer yeast DNA microarray. The signal was detected using GCOS; GeneChip Operating Software 1.0 (manufactured by Affymetrix Co.). Expression pattern of non-ScCTA1 gene is shown in Figure 3. As a result, it was confirmed that non-ScCTA1 gene was expressed in the general beer fermentation.

### Example 3: Preparation of non-ScCTA1 Gene-Highly Expressed Strain

The non-ScCTA1/pCR2.1-TOPO described in Example 1 was digested with restriction enzymes SacI and NotI to prepare a DNA fragment including non-ScCTA1 gene. This fragment was linked to pUP3GLP2 treated with restriction enzymes SacI and NotI, thereby constructing a non-ScCTA1 high expression vector, pUP-nonScCTA1. The yeast expression vector, pUP3GLP2, is a YIp type (chromosome integration type) yeast expression vector having orotidine-5-phosphoric acid decarboxylase gene URA3 at the homologous recombinant site. The introduced gene was highly expressed by the promoter and terminator of glycerylaldehyde-3-phosphoric acid dehydrogenase gene, TDH3. Drug-resistant gene YAP1 as a selective marker for yeast was introduced under the control of the promoter and terminator of galactokinase GAL1, whereby the expression is induced in a culture media comprising galactose. Ampicillin-resistant gene Amp^{r} as a selective marker for *E. coli* was also included.

Using the high expression vector prepared by the above method, *Saccharomyces pastorianus* Weihenstephan 164 strain was transformed by to the method described in Japanese Patent Application Laid-Open No. 07-303475. A cerulenin-resistant strain was selected in a YPGal plate medium (1% yeast extract, 2% polypeptone, 2% galactose, 2% agar) containing 1.0 mg/L of cerulenin.

### Example 4: Analysis of Amount of Sulfite Produced during Beer Fermentation

The parent strain and non-ScCTA1-highly expressed strain obtained in Example 3 were used to carry out fermentation test under the following conditions.

| | |
|---|---|
| Wort extract concentration | 12.87% |
| Wort content | 2 L |
| Wort dissolved oxygen concentration | approximately 8 ppm |
| Fermentation temperature | 15°C, constant |
| Yeast pitching rate | 10.5 g wet yeast cells/2L Wort |

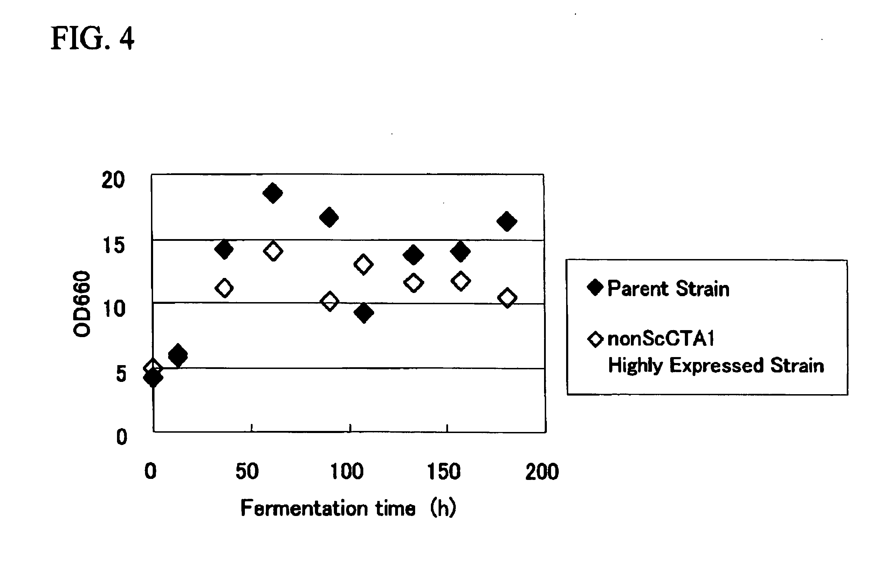

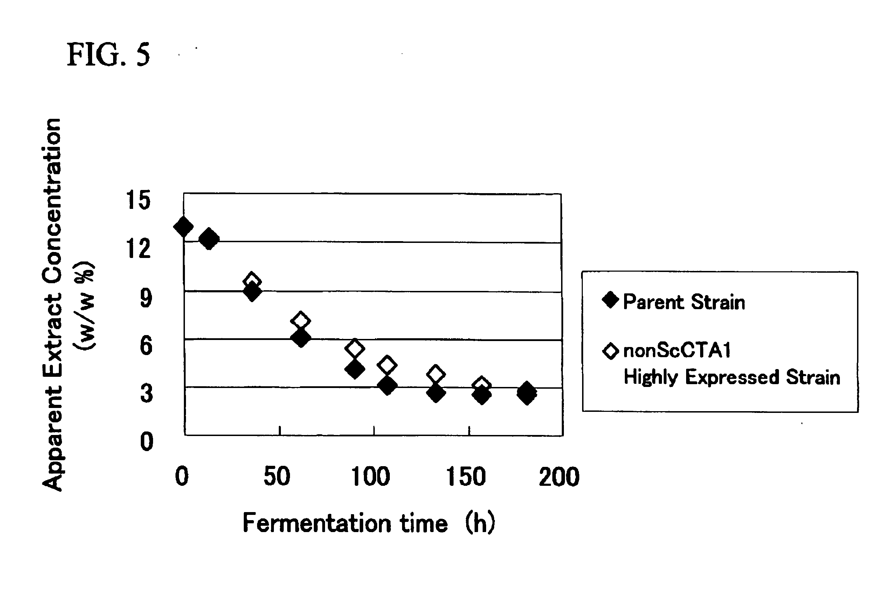

The fermentation broth was sampled with time to observe the cell growth (OD660) (Fig. 4) and extract consumption with time (Fig. 5). Quantification of sulfite concentration at completion of fermentation was carried out by collecting sulfite into hydrogen peroxide aqueous solution by distillation under acidic condition, and titration with alkali (Revised BCOJ Beer Analysis Method by the Brewing Society of Japan).

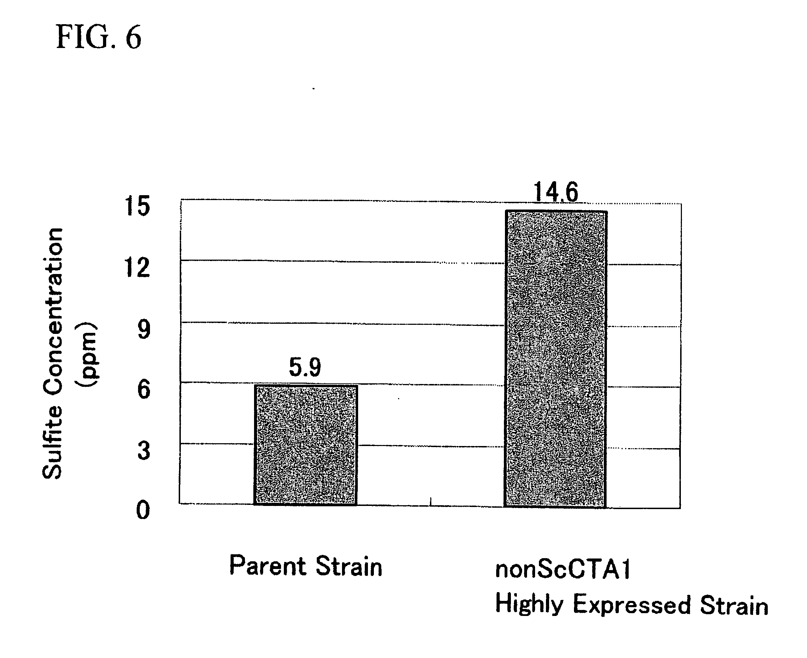

As shown in Fig. 6, the non-ScCTA1-highly expressed strain produced sulfite approximately 2.5 times greater than the parent strain. In addition, significant differences were not observed between the parent strain and the highly expressed strain in cell growth and extract consumption in this testing.

### Reference Example 5: Cloning of Gene Encoding Catalase (ScCTA1)

A gene encoding a catalase (ScCTA1 gene; SEQ ID NO: 3) specific to a lager brewing yeast was found, as a result of a search utilizing the comparison database described in Japanese Patent Application Laid-Open No. 2004-283169. Based on the acquired nucleotide sequence information, primers ScCTA1_for (SEQ ID NO: 7) and ScCTA1_rv (SEQ ID NO: 8) were designed to amplify the full-length genes, respectively. PCR was carried out using chromosomal DNA of a genome sequencing strain, *Sacharomyces pastorianus* Weihenstephan 34/70 strain, as a template to obtain DNA fragments including the full-length gene of ScCTA1.

The thus-obtained ScCTA1 gene fragment was inserted into pCR2.1-TOPO vector (manufactured by Invitrogen Corporation) by TA cloning. The nucleotide sequences of ScCTA1 gene were analyzed according to Sangers method (F. Sanger, Science, 214: 1215, 1981) to confirm the nucleotide sequence.

### Reference Example 6: Analysis of Expression of ScCTA1 Gene during Beer Fermentation

A beer fermentation test was conducted using a lager brewing yeast, *Saccharomyces pastorianus* 34/70 strain and then mRNA extracted from yeast cells during fermentation was analyzed by a yeast DNA microarray.

| | |
|---|---|
| Wort extract concentration | 12.69% |
| Wort content | 70 L |
| Wort dissolved oxygen concentration | 8.6 ppm |
| Fermentation temperature | 15°C |
| Yeast pitching rate | 12.8×10⁶ cells/mL |

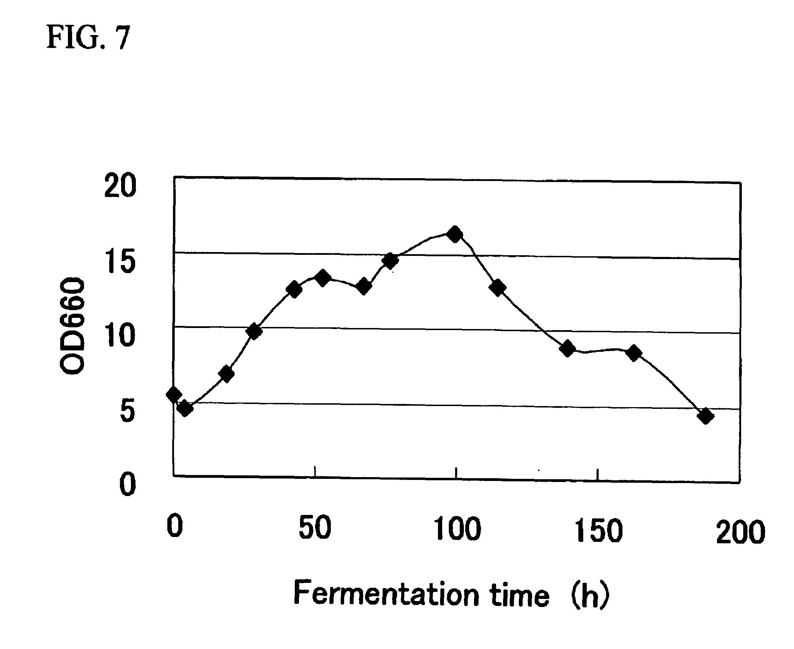

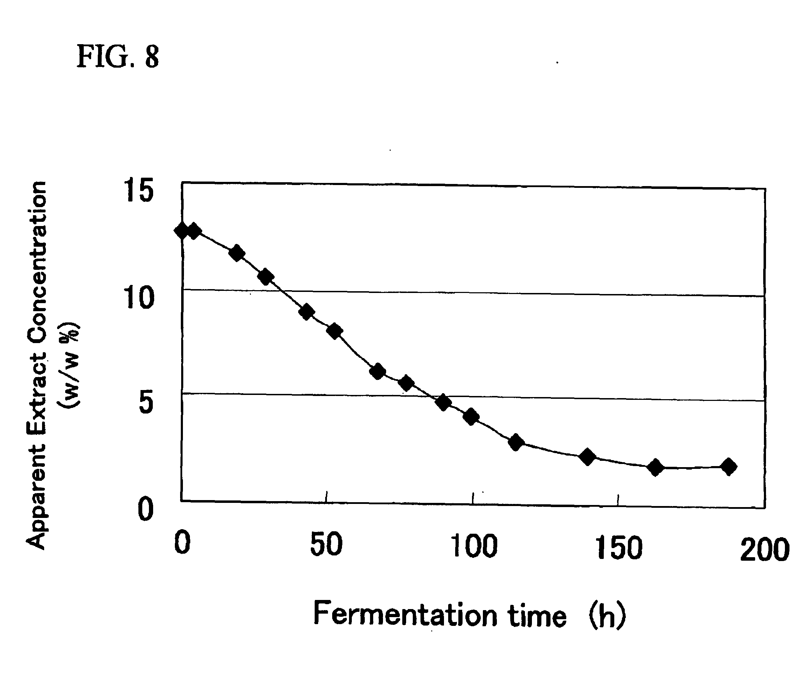

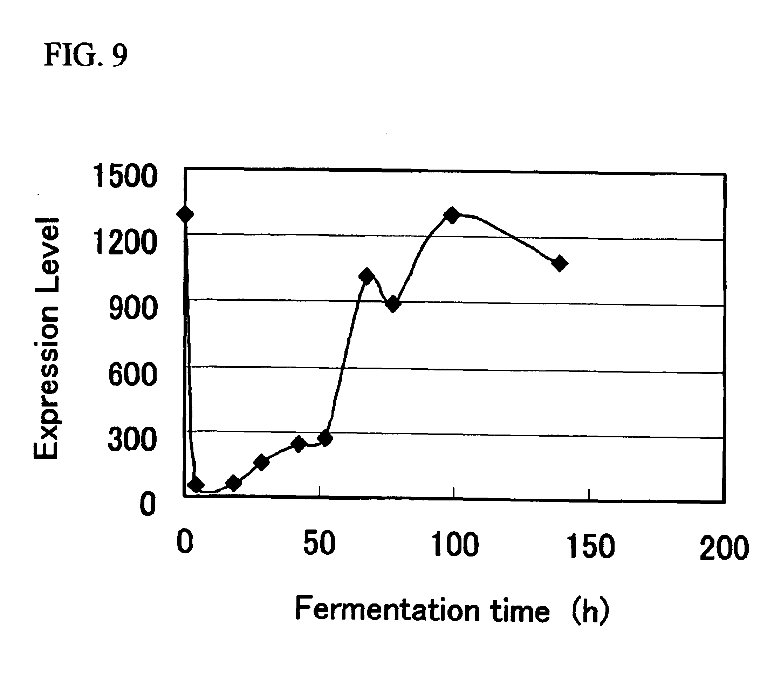

Sampling of fermentation liquid was performed with time, and variation with time of yeast growth amount (Fig. 7) and apparent extract concentration (Fig. 8) was observed. Simultaneously, yeast cells were sampled to prepare mRNA, and the prepared mRNA was labeled with biotin and was hybridized to a beer yeast DNA microarray. The signal was detected using GCOS; GeneChip Operating Software 1.0 (manufactured by Affymetrix Co.). Expression pattern of ScCTA1 gene is shown in Figure 9. As a result, it was confirmed that ScCTA1 gene was expressed in the general beer fermentation.

### Reference Example 7: Preparation of ScCTA1-Highly Expressed Strain

The ScCTA1/pCR2.1-TOPO described in Example 5 was digested with restriction enzymes SacI and NotI to prepare a DNA fragment including ScCTA1 gene. This fragment was linked to pUP3GLP2 treated with restriction enzymes SacI and NotI, thereby constructing a ScCTA1 high expression vector, pUP-ScCTA1.

Using the high expression vector prepared by the above method, *Saccharomyces pasteurianus* Weihenstephaner 164 strain was transformed by the method described in Japanese Patent Application Laid-open No. H7-303475. A cerulenin-resistant strain was selected in a YPGal plate medium (1% yeast extract, 2% polypeptone, 2% galactose, 2% agar) containing 1.0 mg/L of cerulemn.

### Reference Example 8: Analysis of Amount of Sulfite Produced during Beer Fermentation

The parent strain and ScCTA1-highly expressed strain obtained in Example 7, are used to carry out fermentation test under the following conditions.

| | |
|---|---|
| Wort extract concentration | 12.87% |
| Wort content | 2 L |
| Wort dissolved oxygen concentration | approximately 8 ppm |
| Fermentation temperature | 15°C, constant |
| Yeast pitching rate | 10.5 g wet yeast cells/2L Wort |

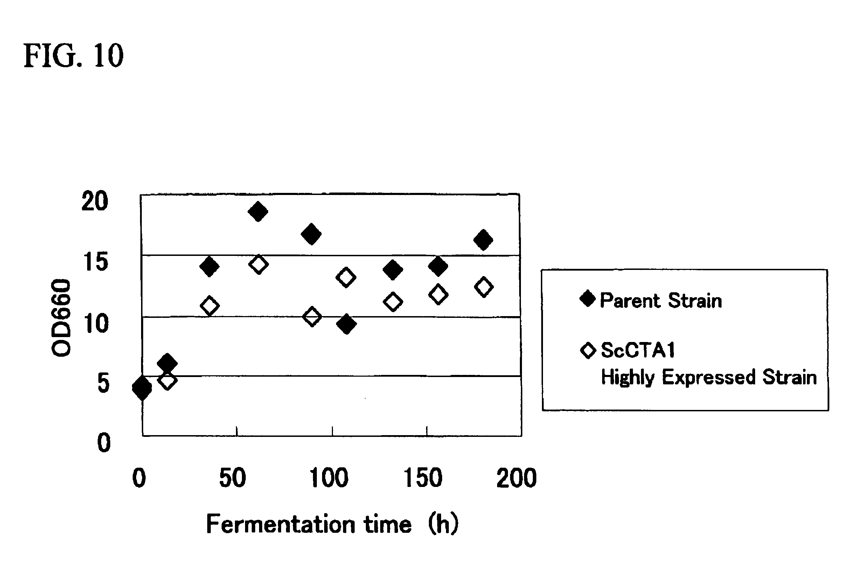

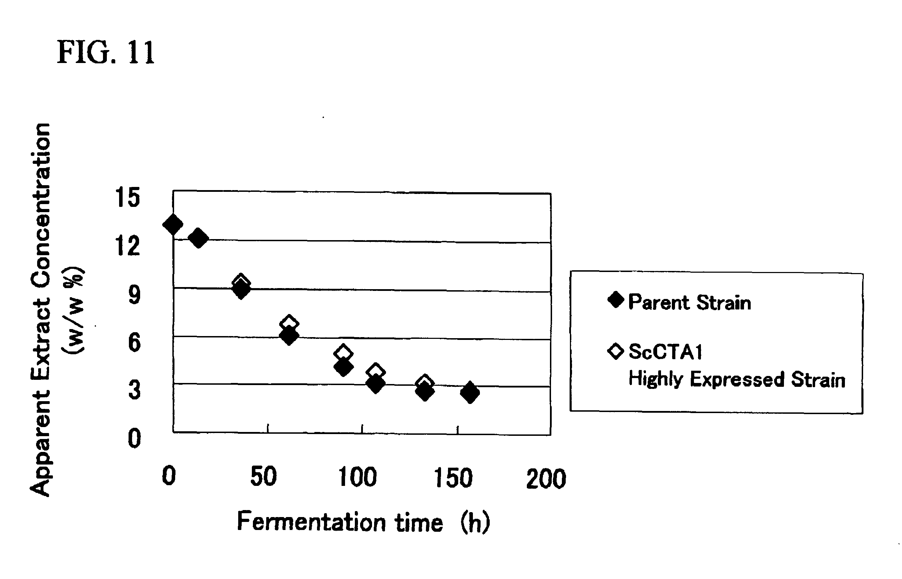

The fermentation broth was sampled with time to observe the cell growth (OD660) (Fig. 10) and extract consumption with time (Fig. 11). Quantification of sulfite concentration at completion of fermentation was carried out by collecting sulfite into hydrogen peroxide aqueous solution by distillation under acidic condition, and titration with alkali (Revised BCOJ Beer Analysis Method by the Brewing Society of Japan).

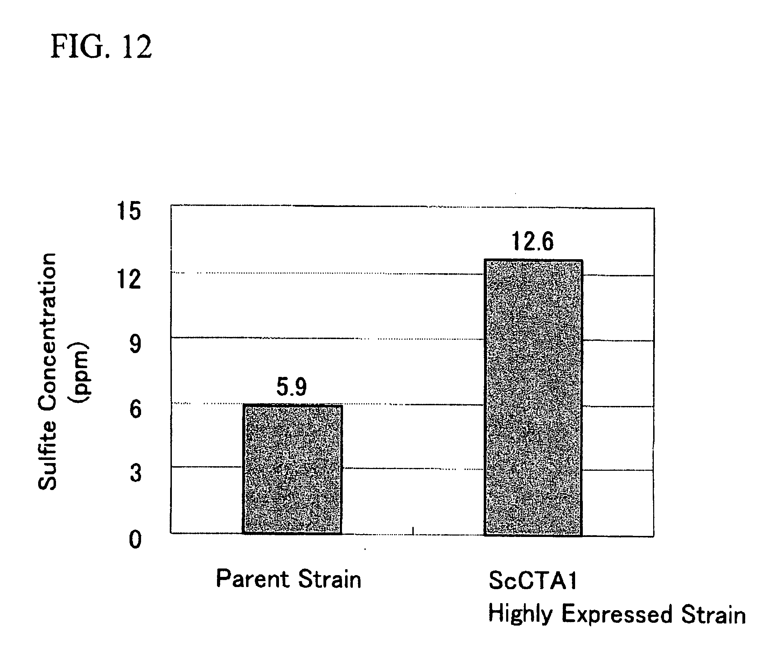

As shown in Fig. 12, the ScCTA1-highly expressed strain produced sulfite approximately 2.1 times greater than the parent strain. In addition, significant differences were not observed between the parent strain and the highly expressed strain in cell growth and extract consumption in this testing.

### INDUSTRIAL APPLICABILITY

According to the method for producing alcoholic beverages of the present invention, because of increase in amount of sulfite which has an anti-oxidative effect in products, alcoholic beverages with superior flavor stability and longer shelf life can be produced.

## Claims

1. A polynucleotide selected from the group consisting of:
(a) a polynucleotide comprising a polynucleotide consisting of the nucleotide sequence of SEQ ID NO:1;
(b) a polynucleotide comprising a polynucleotide encoding a protein consisting of the amino acid sequence of SEQ ID NO:2;
(c) a polynucleotide comprising a polynucleotide encoding a protein consisting of the amino acid sequence of SEQ ID NO:2 with one to 5 amino acids thereof being deleted, substituted, inserted and/or added, having a catalase activity and leading to an increase in the production of sulfite when expressed in yeast; and
(d) a polynucleotide comprising a polynucleotide encoding a protein having an amino acid sequence having 99% or higher identity with the amino acid sequence of SEQ ID NO:2, having a catalase activity and leading to an increase in the production of sulfite when expressed in yeast.

2. The polynucleotide of Claim 1 comprising a polynucleotide consisting of SEQ ID NO: 1.

3. The polynucleotide of Claim 1 comprising a polynucleotide encoding a protein consisting of SEQ ID NO: 2.

4. The polynucleotide of any one of Claims 1 to 3, wherein the polynucleotide is DNA.

5. A protein encoded by the polynucleotide of any one of Claims 1 to 4.

6. A vector comprising the polynucleotide of any one of Claims 1 to 4.

7. A yeast, wherein the vector of Claim 6 is introduced.

8. The yeast of Claim 7, wherein a sulfite-producing capability is enhanced.

9. The yeast of Claim 8, wherein a sulfite-producing capability is enhanced by increasing an expression level of the protein of Claim 5.

10. A method for producing an alcoholic beverage comprising culturing the yeast of any one of Claims 7 to 9.

11. The method for producing an alcoholic beverage of Claim 10, wherein the brewed alcoholic beverage is a malt beverage.

12. The method for producing an alcoholic beverage of Claim 10, wherein the brewed alcoholic beverage is wine.

13. A method for assessing a test yeast for its sulfite-producing capability, comprising using a primer or a probe designed based on a nucleotide sequence of a gene having the nucleotide sequence of SEQ ID NO: 1.

14. A method for assessing a test yeast for its sulfite-producing capability, comprising: culturing a test yeast; and measuring an expression level of a gene having the nucleotide sequence of SEQ ID NO: 1.

15. A method for selecting a yeast, comprising: culturing a reference yeast and test yeasts; measuring an expression level of a gene having the nucleotide sequence of SEQ ID NO: 1 in each yeast; and selecting a test yeast having the gene expressed higher than that in the reference yeast.

16. A method for selecting a yeast, comprising: culturing a reference yeast and test yeasts; quantifying the protein according to Claim 5 in each yeast; and selecting a test yeast having said protein in a larger amount than that in the reference yeast.

17. A method for producing an alcoholic beverage comprising: conducting fermentation for producing an alcoholic beverage using the yeast according to any one of Claims 7 to 9; and adjusting sulfite concentration.

## Patentansprüche

1. Polynucleotid, das ausgewählt ist aus der Gruppe bestehend aus:
(a) einem Polynucleotid umfassend ein Polynucleotid, welches aus der Nucleotidsequenz der SEQ ID NO: 1 besteht;
(b) einem Polynucleotid umfassend ein Polynucleotid, das ein Protein codiert, welches aus der Aminosäuresequenz der SEQ ID NO: 2 besteht;
(c) einem Polynucleotid umfassend ein Polynucleotid, das ein Protein codiert, welches aus der Aminosäuresequenz der SEQ ID NO: 2 besteht, in welcher eine bis 5 Aminosäuren deletiert, substituiert, inseriert und/oder hinzugefügt wurden, eine Katalase-Aktivität hat und, wenn in Hefe exprimiert, zu einer Zunahme in der Herstellung von Sulfit führt; und
(d) einem Polynucleotid umfassend ein Polynucleotid, welches ein Protein codiert, welches eine Aminosäuresequenz mit 99%iger oder höherer Identität mit der Aminosäuresequenz der SEQ ID NO: 2 hat, eine Katalase-Aktivität hat und, wenn in Hefe exprimiert, zu einer Zunahme in der Herstellung von Sulfit führt.

2. Polynucleotid nach Anspruch 1, umfassend ein Polynucleotid, welches aus der SEQ ID NO: 1 besteht.

3. Polynucleotid nach Anspruch 1, umfassend ein Polynucleotid, das ein Protein codiert, welches aus der SEQ ID NO: 2 besteht.

4. Polynucleotid nach einem der Ansprüche 1 bis 3, wobei das Polynucleotid DNA ist.

5. Protein, welches durch das Polynucleotid nach einem der Ansprüche 1 bis 4 codiert wird.

6. Vektor, welcher das Polynucleotid nach einem der Ansprüche 1 bis 4 umfasst.

7. Hefe, in welche der Vektor nach Anspruch 6 eingeführt ist.

8. Hefe nach Anspruch 7, in der eine Sulfit-produzierende Fähigkeit verstärkt ist.

9. Hefe nach Anspruch 8, in der eine Sulfit-produzierende Fähigkeit durch eine Zunahme des Expressionsspiegels des Proteins nach Anspruch 5 verstärkt ist.

10. Verfahren zur Herstellung eines alkoholischen Getränks, umfassend die Züchtung der Hefe nach einem der Ansprüche 7 bis 9.

11. Verfahren zur Herstellung eines alkoholischen Getränks nach Anspruch 10, wobei das gebraute alkoholische Getränk ein Malzgetränk ist.

12. Verfahren zur Herstellung eines alkoholischen Getränks nach Anspruch 10, wobei das gebraute alkoholische Getränk Wein ist.

13. Verfahren zur Beurteilung einer Test-Hefe bezüglich ihrer Sulfit-produzierenden Fähigkeit, umfassend die Verwendung eines Primers oder einer Sonde, welche(r) basierend auf einer Nucleotidsequenz eines Gens, welches die Nucleotidsequenz der SEQ ID NO: 1 hat, entworfen wurde.

14. Verfahren zur Beurteilung einer Test-Hefe bezüglich ihrer Sulfit-produzierenden Fähigkeit, umfassend: Züchten einer Test-Hefe; und Messen eines Expressionsspiegels eines Gens, welches die Nucleotidsequenz der SEQ ID NO: 1 hat.

15. Verfahren zur Auswahl einer Hefe, umfassend: Züchten einer Referenz-Hefe und von Test-Hefen; Messen eines Expressionsspiegels eines Gens, welches die Nucleotidsequenz der SEQ ID NO: 1 hat, in jeder Hefe; und Auswahl einer Test-Hefe, in welcher das Gen höher als das in der Referenz-Hefe exprimiert wird.

16. Verfahren zur Auswahl einer Hefe, umfassend: Züchten einer Referenz-Hefe und von Test-Hefen; Quantifizieren des Proteins gemäß Anspruch 5 in jeder Hefe; und Auswahl einer Test-Hefe, welche das Protein in einer größeren Menge als das in der Referenz-Hefe hat.

17. Verfahren zur Herstellung eines alkoholischen Getränks, umfassend: Ausführen einer Fermentation zur Produktion eines alkoholischen Getränks unter Verwendung der Hefe gemäß einem der Ansprüche 7 bis 9; und Anpassen der Sulfitkonzentration.

## Revendications

1. Polynucléotide choisi parmi le groupe consistant en :
(a) un polynucléotide comprenant un polynucléotide consistant en la séquence nucléotidique de SEQ ID NO: 1 ;
(b) un polynucléotide comprenant un polynucléotide codant une protéine consistant en la séquence d'acides aminés de SEQ ID NO: 2 ;
(c) un polynucléotide comprenant un polynucléotide codant une protéine consistant en la séquence d'acides aminés de SEQ ID NO: 2 avec un à cinq acides aminés de celle-ci étant supprimés, substitués, insérés et/ou ajoutés, ayant une activité catalase et conduisant à une augmentation de la production de sulfite lorsqu'il est exprimé dans la levure ; et
(d) un polynucléotide comprenant un polynucléotide codant une protéine ayant une séquence d'acides aminés ayant 99% ou plus d'identité avec la séquence d'acides aminés de SEQ ID NO: 2, ayant une activité catalase et conduisant à une augmentation de la production de sulfite lorsqu'il est exprimé dans la levure.

2. Polynucléotide de la revendication 1 comprenant un polynucléotide consistant en SEQ ID NO: 1.

3. Polynucléotide de la revendication 1 comprenant un polynucléotide codant une protéine consistant en SEQ ID NO: 2.

4. Polynucléotide de l'une quelconque des revendications 1 à 3, ledit polynucléotide étant de l'ADN.

5. Protéine codée par le polynucléotide de l'une quelconque des revendications 1 à 4.

6. Vecteur comprenant le polynucléotide de l'une quelconque des revendications 1 à 4.

7. Levure dans laquelle le vecteur de la revendication 6 est introduit.

8. Levure de la revendication 7, dans laquelle une capacité de production de sulfite est améliorée.

9. Levure de la revendication 8, dans laquelle une capacité de production de sulfite est améliorée en augmentant un niveau d'expression de la protéine de la revendication 5.

10. Méthode pour produire une boisson alcoolisée comprenant la culture de la levure de l'une quelconque des revendications 7 à 9.

11. Méthode pour produire une boisson alcoolisée de la revendication 10, dans laquelle la boisson alcoolisée brassée est une boisson à base de malt.

12. Méthode pour produire une boisson alcoolisée de la revendication 10, dans laquelle la boisson alcoolisée brassée est du vin.

13. Méthode pour évaluer une levure de test pour sa capacité de production de sulfite, comprenant l'utilisation d'une amorce ou d'une sonde conçue sur la base d'une séquence nucléotidique d'un gène ayant la séquence nucléotidique de SEQ ID NO: 1.

14. Méthode pour évaluer une levure de test pour sa capacité de production de sulfite, comprenant : la culture d'une levure de test ; et la mesure d'un niveau d'expression d'un gène ayant la séquence nucléotidique de SEQ ID NO: 1.

15. Méthode pour sélectionner une levure, comprenant : la culture d'une levure de référence et de levures de test ; la mesure d'un niveau d'expression d'un gène ayant la séquence nucléotidique de SEQ ID NO: 1 dans chaque levure ; et la sélection d'une levure de test ayant le gène exprimé plus fortement que dans la levure de référence.

16. Méthode pour sélectionner une levure, comprenant : la culture d'une levure de référence et de levures de test ; la quantification de la protéine selon la revendication 5 dans chaque levure ; et la sélection d'une levure de test ayant ladite protéine dans une plus grande quantité que celle dans la levure de référence.

17. Méthode pour produire une boisson alcoolisée comprenant : la conduite d'une fermentation pour produire une boisson alcoolisée en utilisant la levure selon l'une quelconque des revendications 7 à 9 ; et l'ajustement de la concentration en sulfite.
